# EUROPEAN PATENT SPECIFICATION

(11) **EP 0 556 256 B1**
(45) Date of publication and mention of the grant of the patent: **30.08.1995**
(21) Application number: 91919751.7
(22) Date of filing: 07.11.1991
(51) Int. Cl.: A61K 9/12, A61K 9/72

(54) **AEROSOL MEDICAMENTS**
AEROSOLZUSAMMENSETZUNGEN
MEDICAMENTS EN AEROSOL

(30) Priority: 09.11.1990 GB 9024365
(43) Date of publication of application: 25.08.1993
(73) Proprietor: GLAXO GROUP LIMITED, Greenford, Middlesex UB6 0NN (GB)
(72) Inventor: TAYLOR, Anthony James, Ware, Hertfordshire SG12 0DG (GB); BURNELL, Patricia Kwong Phieu, Ware, Hertfordshire SG12 0DG (GB)
(74) Representative: Towler, Philip Dean
(86) International application number: GB9101960
(87) International publication number: WO9208446

(56) References cited:
- EP-A- 0 372 777
- WO-A-91/04011
- US-A- 4 352 789

## Description

This invention relates to aerosol formulations of use in the administration of medicaments by inhalation.

The use of aerosols to administer medicaments has been known for several decades. Such aerosols generally comprise the medicament, one or more chlorofluorocarbon propellants and either a surfactant or a solvent, such as ethanol.

The most commonly used aerosol propellants for medicaments have been Freon 11 (CCl₃F) in admixture with Freon 12 (CCl₂F₂) and Freon 114 (CF₂Cl.CF₂Cl). However these propellants are now believed to provoke the degradation of stratospheric ozone and there is thus a need to provide aerosol formulations for medicaments which employ so called "ozone-friendly" propellants.

A class of propellants which are believed to have minimal ozone-depleting effects in comparison to conventional chlorofluorocarbons comprise hydrogen-containing chlorofluorocarbons and fluorocarbons; medicinal aerosol formulations using such propellant systems are disclosed in, for example, EP 0372777. EP 0372777 requires the use of 1,1,1,2-tetrafluoroethane in combination with both a cosolvent having greater polarity than 1,1,1,2-tetrafluoroethane (e.g. an alcohol or a lower alkane) and a surfactant in order to achieve a stable formulation of a medicament powder. In particular it is noted in the specification at page 3, line 7 that "it has been found that the use of Propellant 134a (1,1,1,2-tetrafluoroethane) and drug as a binary mixture or in combination with a conventional surfactant such as sorbitan trioleate does not provide formulations having suitable properties for use with pressurised inhalers".

We have now surprisingly found that, in contradistinction to this teaching, it is in fact possible to obtain stable dispersions of finely-powdered medicaments together with surfactants in hydrogen-containing fluorocarbon or chlorofluorocarbon propellants such as 1,1,1,2-tetrafluoroethane if the surfactant is present as a dry coating on the particles of medicament and that the stability of the resulting dispersion is enhanced by the presence of small quantities (e.g. up to 5% w/w) of cosolvents having higher polarity than the propellant such as lower alkanols (e.g. ethanol). This is in contrast to the procedure of EP 0372777, where the medicament and surfactant are simultaneously homogenised, e.g. in ethanol, prior to addition of the propellant.

There is thus provided an aerosol formulation comprising:-
(A) a medicament in particulate form said medicament having a particle size of less than 100 »m and having a surface coating of a surfactant, which surfactant has no affinity for said propellant.
(B) a hydrogen-containing fluorocarbon or chlorofluorocarbon propellant; and
(C) a cosolvent having higher polarity than the propellant which cosolvent is present in an amount of up to 5% w/w based upon propellant.

The propellants for use in the invention may be any hydrogen-containing fluorocarbon or chlorofluorocarbon or mixtures thereof having a sufficient vapour pressure to render them effective as propellants. Such propellants include for example C₁₋₄hydrogen-containing fluorocarbons and chlorofluorocarbons such as CH₂ClF, CClF₂-CHClF, CF₃-CHClF, CHF₂-CClF₂, CHClF-CHF₂, CF₃-CH₂Cl, CHF₂-CHF₂, CF₃-CH₂F, CClF₂-CH₃, CHF₂-CH₃ and CF₃CHFCF₃.

Where mixtures of the hydrogen-containing fluorocarbons or chlorofluorocarbons are employed they may be mixtures of the above identified compounds or mixtures, preferably binary mixtures, with other hydrogen-containing fluorocarbons or chlorofluorocarbons for example CHClF₂, CH₂F₂ and CF₃CH₃.

The propellant may additionally contain a volatile saturated hydrocarbon for example n-butane, isobutane, pentane and isopentane. Preferably a single hydrogen-containing fluorocarbon or chlorofluorocarbon is employed as the propellant. Preferably the propellant will be a non-solvent for the medicament. Particularly preferred as propellants are 1,1,1,2-tetrafluoroethane (CF₃.CH₂F) and 1,1,1,2,3,3,3-heptafluoro-n-propane (CF₃.CHF.CF₃).

It is desirable that the formulations of the invention contain no components which may provoke the degradation of stratospheric ozone. In particular it is desirable that the formulations are substantially free of chlorofluorocarbons such as CCl₃F, CCl₂F₂ and CF₃CCl₃.

Cosolvents which may be used include (e.g. C₂₋₆) aliphatic alcohols and polyols such as ethanol, ispropanol and propylene glycol. Preferably ethanol will be employed. In general only small quantities (e.g. 0.05 - 3.0% w/w) are required to improve the stability of the dispersion - the use of quantities in excess of 5% w/w may tend to dissolve the medicament and/or surfactant. Formulations in accordance with the invention preferably contain less than 1% w/w, e.g. about 0.1% w/w, of cosolvent.

Medicaments which may be administered using aerosol formulations according to the invention include any drugs useful in inhalation therapy which may be presented in a form which is substantially completely insoluble in the selected propellant. Appropriate medicaments may thus be selected from, for example, analgesics, e.g. codeine, dihydromorphine, ergotamine, fentanyl or morphine; anginal preparations, e.g. dilitiazem; antiallergics, e.g. cromolyn; antibiotics, e.g. cephalosporins, penicillins, streptomycin, sulphonamides or tetracyclines; antihistamines, e.g. methapyrilene; anti-inflammatories, e.g. beclomethasone, flunisolide or fluticasone; antitussives, e.g. noscapine; bronchodilators, e.g. ephedrine, epinephrine, isoproterenol, metaproterenol, phenylephrine, phenylpropanolamine, pirbuterol, rimiterol, salbutamol, salmeterol, (-)-4-amino-3,5-dichloro-α-[[[6-[2-(2-pyridinyl)ethoxy]hexyl]amino] methyl]benzenemethanol or terbutaline; diuretics, e.g. amiloride; hormones, e.g. cortisone, hydrocortisone or prednisolone; and therapeutic proteins and peptides, e.g. glucagon. Where appropriate the medicaments may be used in the form of salts (e.g as alkali metal or amine salts or as acid addition salts) or as esters (e.g. lower alkyl esters) to optimise the activity and/or stability of the medicament and/or to minimise the solubility of the medicament in the propellant.

Particularly preferred medicaments for administration using aerosol formulations in accordance with the invention include bronchodilators and anti-inflammatory steroids currently used in the treatment of asthma by inhalations therapy. Salbutamol (eg. as the sulphate), salmeterol (e.g. as the hydroxynaphthoate), beclomethasone esters (e.g. the dipropionate) or fluticasone esters (e.g. the propionate) are especially preferred medicaments for use in the formulations of the invention.

The surfactants for use in the invention will have no affinity for the propellant (that is to say they will contain no groups which have affinity with the propellant).

The surfactants must be physiologically acceptable upon administration by inhalation. Surfactants within this category include materials such as benzalkonium chloride, lecithin, oleic acid and sorbitan trioleate (Span ^{R} 85).

The use of substantially non-ionic surfactants which have reasonable solubility in substantially non-polar solvents is frequently advantageous since it facilitates coating of the medicament particles using solutions of surfactant in non-polar solvents in which the medicament has limited or minimal solubility.

Thus according to a further aspect of the invention the aerosol formulations may be prepared by slurrying particulate (e.g. micronised) medicament with a solution of a surfactant such as lecithin in a substantially non-polar solvent (e.g. a lower alkane such as isopentane or a chlorofluorocarbon such as trichlorofluoromethane), optionally homogenising the slurry (e.g. by sonication), removing the solvent and if necessary simultaneously and/or subsequently breaking up the resulting solid cake, and dispersing the thus-obtained surfactant-coated particulate medicament in the chosen propellant in an appropriate aerosol container, e.g. with the aid of sonication. It may be preferred to add the cosolvent after the coated medicament and propellant have been combined, in order to minimise any solubilising effects of the cosolvent and thereby enhance the stability of the dispersion. The process is desirably carried out under anhydrous conditions to obviate hydrate formation.

The particle size of the finely-powdered medicament should be such as to permit inhalation of substantially all of the medicament into the bronchial system upon administration of the aerosol formulation and will thus be less than 100 »m, desirably less than 20 »m, and preferably in the range 2-10 »m, e.g. 2-5 »m.

The amount of surfactant employed in coating the particulate medicament is desirably in the range 0.01-10.0% w/w, preferably 0.05-5.0% w/w, relative to the medicament, and may advantageously be chosen such that a substantially monomolecular coating of surfactant is formed. The final aerosol formulation desirably contains 0.005-5.0% w/w, preferably 0.01-1.0% w/w, of coated medicament relative to the total weight of the formulation.

The following non-limitative Examples serve to illustrate the invention.

### EXAMPLE 1

### (A) Preparation of Lecithin-coated Salmeterol Hydroxynapthoate

(a) Lecithin (Epikuron 145V - 3.65mg) was dissolved in a small amount of isopentane and the resulting solution was added to micronised salmeterol hydroxynaphthoate (0.5g). Further isopentane (7.0g total) was added to form a slurry, which was sonicated for 3 minutes. The resulting suspension was dried by evaporating the isopentane at ambient temperature in a fume cupboard, whereafter the resulting dried plug was roughly broken up and then dried further in a vacuum oven. The thus-obtained product was further broken up using a mortar and pestle to yield lecithin-coated salmeterol hydroxynaphthoate containing 0.73% w/w of lecithin relative to the salmeterol hydroxynaphthoate.
(b) The above procedure was repeated except that 6.10mg of lecithin was employed, whereby a coated product containing 1.22% w/w of lecithin relative to the salmeterol hydroxynaphthoate was obtained.
(c) The above procedure was again repeated except that 7.80mg of lecithin was employed, thereby yielding a coated product containing 1.56% w/w of lecithin relative to the salmeterol hydroxynaphthoate.

### (B) Formulation of Lecithin-coated Salmeterol Hydroxynaphthoate in Ethanol-containing 1,1,1,2-Tetrafluoroethane

(a) Samples of each of the products of Examples 1(A) (a)-(c) (9.1mg) were weighed into aerosol cans containing dried ethanol (0.018g - 0.1% w/w of total fill weight) and 1,1,1,2-tetrafluoroethane (ca. 5g). Further 1,1,1,2-tetrafluoroethane (total 18.2g - 99.95% w/w of total fill weight) was added to each can, whereafter suitable metering valves were crimped onto the cans, which were then each sonicated for 5 minutes. The resulting aerosols contained salmeterol in an amount equivalent to 240 actuations at 25 »g per actuation.
(b) The above procedure was repeated except that 0.091g of dried ethanol was employed in each formulation, whereby aerosols containing 0.5% w/w ethanol and 99.45% w/w of propellant were obtained.

## Claims

1. An aerosol formulation comprising:-
(A) a hydrogen-containing fluorocarbon or chlorofluorocarbon propellant;
(B) a cosolvent having higher polarity than said propellant, which cosolvent is present in an amount of up to 5% w/w based upon propellant; and
(C) a medicament in particulate form said medicament having a particle size of less than 100 »m and having a surface coating of a surfactant, which surfactant has no affinity for said propellant.

2. A formulation as claimed in claim 1 wherein the propellant comprises 1,1,1,2-tetrafluoroethane or 1,1,1,2,3,3,3-heptafluoropropane.

3. A formulation as claimed in claim 1 wherein the propellant comprises 1,1,1,2-tetrafluoroethane.

4. A formulation as claimed in any one of claims 1 to 3 which is substantially free of chlorofluorocarbons.

5. A formulation as claimed in any one of claims 1 to 4 wherein the cosolvent is selected from an aliphatic alcohol or a polyol.

6. A formulation as claimed in any one of claims 1 to 5 wherein the surfactant is present in an amount of from 0.01 to 10% w/w based on the medicament.

7. A formulation as claimed in any one of claims 1 to 6 wherein the surfactant is selected from benzalkonium chloride, lecithin, oleic acid and sorbitan trioleate.

8. A formulation as claimed in any one of claims 1 to 7 wherein the surfactant-coated medicament is present in an amount of 0.005-5% w/w based upon the total weight of the formulation.

9. A formulation as claimed in any one of claims 1 to 8 wherein the medicament is selected from salbutamol, salmeterol, beclomethasone esters or fluticasone esters.

10. A formulation as claimed in any one of claims 1 to 9 wherein the medicament is selected from salbutamol sulphate, salmeterol hydroxynaphthoate, beclomethasone diproprionate or fluticasone propionate.

11. A method for the preparation of an aerosol formulation as claimed in claim 1 comprising dispersing a surface-coated medicament in a hydrogen-containing fluorocarbon or chlorofluorocarbon propellant in an aerosol container and then adding the cosolvent.

12. A method as claimed in claim 11 wherein the surface-coated medicament is obtained by slurrying particulate medicament with a solution of a surfactant in a substantially non-polar solvent and then removing the solvent.

## Patentansprüche

1. Aerosolzubereitung, enthaltend
(A) ein Wasserstoff-enthaltendes Fluorkohlenwasserstoff- oder Chlorfluorkohlenwasserstoff-Treibmittel;
(B) ein Co-Lösungsmittel mit einer höheren Polarität als das Treibmittel, wobei das Co-Lösungsmittel in einer Menge von bis zu 5% Gew./Gew., bezogen auf das Treibmittel, vorhanden ist; und
(C) ein Arzneimittel in teilchenförmiger Form, wobei das Arzneimittel eine Teilchengröße von weniger als 100 »m und einen Oberflächenüberzug eines oberflächenaktiven Mittels aufweist, wobei das oberflächenaktive Mittel gegenüber dem Treibmittel keine Affinität besitzt.

2. Zubereitung nach Anspruch 1, dadurch **gekennzeichnet,** daß das Treibmittel 1,1,1,2-Tetrafluorethan oder 1,1,1,2,3,3,3-Heptafluorpropan umfaßt.

3. Zubereitung nach Anspruch 1, dadurch **gekennzeichnet,** daß das Treibmittel 1,1,1,2-Tetrafluorethan umfaßt.

4. Zubereitung nach einem der Ansprüche 1 bis 3, dadurch **gekennzeichnet,** daß sie im wesentlichen von Chlorfluorkohlenwasserstoff-Verbindungen frei ist.

5. Zubereitung nach einem der Ansprüche 1 bis 4, dadurch **gekennzeichnet,** daß das Co-Lösungsmittel aus einem aliphatischen Alkohol oder einem Polyol ausgewählt ist.

6. Zubereitung nach einem der Ansprüche 1 bis 5, dadurch **gekennzeichnet,** daß das oberflächenaktive Mittel in einer Menge von 0,01 bis 10% Gew./Gew., bezogen auf das Arzneimittel, vorliegt.

7. Zubereitung nach einem der Ansprüche 1 bis 6, dadurch **gekennzeichnet,** daß das oberflächenaktive Mittel aus Benzalkoniumchlorid, Lecithin, Ölsäure und Sorbitantrioleat ausgewählt ist.

8. Zubereitung nach einem der Ansprüche 1 bis 7, dadurch **gekennzeichnet,** daß das mit einem oberflächenaktiven Mittel beschichtete Arzneimittel in einer Menge von 0,005 bis 5% Gew./Gew., bezogen auf das Gesamtgewicht der Zubereitung, vorhanden ist.

9. Zubereitung nach einem der Ansprüche 1 bis 8, dadurch **gekennzeichnet,** daß das Arzneimittel aus Salbutamol, Salmeterol, Beclomethasonestern oder Fluticasonestern ausgewählt ist.

10. Zubereitung nach einem der Ansprüche 1 bis 9, dadurch **gekennzeichnet,** daß das Arzneimittel aus Salbutamolsulfat, Salmeterolhydroxynaphthoat, Beclomethasondipropionat oder Fluticasonpropionat ausgewählt ist.

11. Verfahren zur Herstellung einer Aerosolzubereitung nach Anspruch 1, umfassend die Dispergierung eines oberflächenbeschichteten Arzneimittels in einem Wasserstoff-enthaltenden Fluorkohlenwasserstoff- oder Chlorfluorkohlenwasserstoff-Treibmittel in einem Aerosolbehälter und anschließende Zugabe des Co-Lösungsmittels.

12. Verfahren nach Anspruch 11, dadurch **gekennzeichnet,** daß das oberflächenbeschichtete Arzneimittel durch Aufschlämmung des teilchenförmigen Arzneimittels mit einer Lösung eines oberflächenaktiven Mittels in einem im wesentlichen nichtpolaren Lösungsmittel und durch anschließende Entfernung des Lösungsmittels erhalten worden ist.

## Revendications

1. Composition d'aérosol comprenant :
(A) un propulseur constitué d'un fluorocarbone ou d'un chlorofluorocarbone contenant de l'hydrogène,
(B) un cosolvant possédant une polarité supérieure à celle du propulseur, lequel cosolvant est présent en une proportion allant jusqu'à 5% p/p sur base du propulseur, et
(C) un médicament sous forme particulaire, ce médicament possédant un calibre des particules inférieur à 100 »m et possédant un revêtement en surface d'un surfactif, lequel surfactif n'a pas d'affinité pour le propulseur précité.

2. Composition suivant la revendication 1, caractérisée en ce que le propulseur est constitué de 1,1,1,2-tétrafluoréthane ou de 1,1,1,2,3,3,3-heptafluoropropane.

3. Composition suivant la revendication 1, caractérisée en ce que le propulseur est constitué de 1,1,1,2-tétrafluoréthane.

4. Composition suivant l'une quelconque des revendications 1 à 3, caractérisée en ce qu'elle est sensiblement dépourvue de chlorofluorocarbones.

5. Composition suivant l'une quelconque des revendications 1 à 4, caractérisée en ce que le cosolvant est choisi parmi un alcool aliphatique et un polyol.

6. Composition suivant l'une quelconque des revendications 1 à 5, caractérisée en ce que le surfactif est présent en une proportion de 0,01 à 10% p/p sur base du médicament.

7. Composition suivant l'une quelconque des revendications 1 à 6, caractérisée en ce que le surfactif est choisi parmi le chlorure de benzalconium, la lécithine, l'acide oléique et le trioléate de sorbitan.

8. Composition suivant l'une quelconque des revendications 1 à 7, caractérisée en ce que le médicament enrobé de surfactif est présent en une proportion de 0,005 à 5% p/p sur base du poids total de la composition.

9. Composition suivant l'une quelconque des revendications 1 à 8, caractérisée en ce que le médicament est choisi parmi le salbutamol, le salmétérol, des esters de la béclométhasone, ou des esters de la fluticasone.

10. Composition suivant l'une quelconque des revendications 1 à 9, caractérisée en ce que le médicament est choisi parmi le sulfate de salbutamol, l'hydroxynaphtoate de salmétérol, le dipropionate de béclométhasone et le propionate de fluticasone.

11. Procédé de préparation d'une composition d'aérosol suivant la revendication 1, caractérisé en ce que l'on disperse un médicament revêtu en surface dans un propulseur constitué d'un fluorocarbone ou chlorofluorocarbone contenant de l'hydrogène dans un récipient pour aérosol et en ce que l'on ajoute ensuite le cosolvant.

12. Procédé suivant la revendication 11, caractérisé en ce que le médicament revêtu en surface est obtenu en mettant un médicament particulaire en suspension avec une solution d'un surfactif dans un solvant sensiblement apolaire et on chasse ensuite le solvant.
